# EUROPEAN PATENT APPLICATION

(11) **EP 1 552 877 A1**
(43) Date of publication of application: **13.07.2005**
(21) Application number: 03741532.0
(22) Date of filing: 22.07.2003
(51) Int. Cl.: B01D 53/44, A61L 9/14, A61L 9/16, B01D 46/26, B05B 15/12

(54) **DEVICE AND METHOD FOR ELIMINATING ODOR**

(30) Priority: 19.07.2002 JP 2002211333
(71) Applicant: Uegaki, Tateo, Sendai-shi, Miyage 983-0011 (JP)
(72) Inventor: UEGAKI, Tateo, Sendai-shi, Miyagi 983-0011 (JP); YOSHIMURA, Koki, Shinagawa-ku, Tokyo 104-0003 (JP)
(74) Representative: Bannerman, David Gardner
(86) International application number: PCT/JP2003/009271
(87) International publication number: WO 2005/007272

(57) **Abstract**

The present invention relates to a deodorization technique for an operation involving malodor, and aims to provide a deodorization apparatus and a deodorization method enhanced in deodorization efficiency and reduced in initial investment and running cost. The deodorization apparatus includes an intake port 13 for taking in air with malodor generated in a coating booth 1, an injection nozzle 12a for supplying deodorant into the air with deodorant taken in through the intake port 13, an exhaust port 17a for discharging the air taken in through the intake port 13, an exhaust fan 17 for forming an airflow from the intake port 13 to the exhaust port 17a, and a second filter 15 which, prior to exhaust through the exhaust port 17a, removes a malodorous substance from the air with deodorant together with the deodorant.

## Description

### TECHNICAL FIELD

The present invention relates to a deodorization technique for an operation involving malodor, and more specifically to a deodorization apparatus and a deodorization method suitably applicable to a coating booth, etc.

### BACKGROUND ART

As an example of an operation booth where an operation involving malodor is conducted, a coating operation booth (hereinafter referred to as a "coating booth") is known. An example of such a coating booth is disclosed in JP 6-328025 A.

The coating booth disclosed in the above-mentioned publication has, as deodorization equipment, a dust collecting filter for collecting coating mist (a malodorous substance) generated during coating operation, a water spray device for removing fine coating mist that has not been collected by the dust collecting filter, etc.

More specifically, the air in the operation booth is sucked in by an air intake fan to collect coating mist suspended in the operation booth by the dust collecting filter. Then, to remove the fine coating mist in the air that has not been collected by the dust collecting filter, water is sprayed from the water spray device into the air. Through adhesion of the sprayed water to the fine coating mist, the remaining fine coating mist is removed from the air. At the bottom of the booth, there is installed a vessel, and the water mixed with fine coating mist (contaminated water) drips into the vessel and is gathered therein. The contaminated water in the vessel is separated afterwards into water and fine coating mist by using a contaminated water processing apparatus such as a centrifugal separator.

In this way, in the conventional coating booth, coating mist (inclusive of fine coating mist) constituting the malodorous substance is caught by the dust collecting filter or the water spray device to suppress the malodor in the operation booth. The contaminated water mixed with fine coating mist is separated and purified by the contaminated water processing apparatus.

Incidentally, a study made by the inventors of the present invention have revealed various points to be improved regarding the above deodorization method.

First, with regard to the water spray, it is necessary to draw water into the water spray device, install a vessel for receiving the water, and to provide a contaminated water processing apparatus, etc. separately from the coating booth apparatus main body, resulting in high initial equipment cost (initial investment) for the coating booth. Further, it is also necessary to periodically repeat troublesome maintenance operations involving water treatment, such as the cleaning of the vessel and the cleaning of the interior of the contaminated processing apparatus.

Further, while effective in removing fine coating mist, the water spray did not prove so effective in deodorizing a gaseous malodorous substance (e.g., a solvent like thinner). Further, to keep the running cost low, water once sprayed is usually utilized again as spray water, which means as the number of times that the water is used increases, the water itself gradually takes onmalodor.

The present invention has beenmade in view of the above problems in the prior-art technique. It is an obj ect of the present invention to provide a deodorization apparatus and a deodorization method enhanced in deodorizing efficiency and reduced in initial investment and running cost.

### DISCLOSURE OF THE INVENTION

A deodorization apparatus according to the present invention is characterized by including: an intake port for taking in a gas with malodor; a deodorant supply device for supplying deodorant into the gas taken in through the intake port; an exhaust port for discharging the gas taken in through the intake port; an airflow forming device for forming an airflow from the intake port to the exhaust port; and a filter which removes a malodorous substance from the gas with malodor together with the deodorant prior to exhaust through the exhaust port.

In the deodorization apparatus of the present invention, constructed as described above, a gas with malodor is first taken in through the intake port. Then, deodorant is supplied to the gas with malodor by the deodorant supply device. The malodorous substance in the air flows toward the discharge port while adhering to the deodorant, and flows into the filter prior to discharge through the discharge port. The malodorous substance is then collected by the filter together with the deodorant.

That is, in the present invention, the malodorous substance is sent along in an airflow while adhering to the deodorant, and is collected by the filter together with the deodorant. Thus, it is possible to suppress malodor without having to provide any large-scale equipment, such as a vessel and a contaminated water processing apparatus. Further, a stable deodorization efficiency can be maintained by an easy operation of filter replacement. Further, since the malodorous substance is caught by using deodorant, deodorization can be effected efficiently also on a gaseous malodorous substance. There are no particular limitations regarding the deodorant as long as it can be diffused and supplied into a gas, and not only a liquid deodorant but also a powder deodorant can be used.

Further, the deodorization apparatus may be constructed such that the deodorization apparatus includes a booth main body for restraining diffusion of malodor, and that the intake port is open to an interior of the booth main body. With this construction, malodor diffusion is suppressed within the booth main body, and the malodor in the booth main body is deodorized by the deodorization apparatus.

Further, the deodorization apparatus may be constructed to include a moving device for moving a collecting surface of the filter for collecting the deodorant and the malodorous substance. Further, inmoving the collecting surface, the moving device maybe constructed to move a collecting surface with a high collection efficiency toward the airflow.

Further, the moving device may include a moving amount changing means for changing a moving amount of the collecting surface and a collection amount calculating means for calculating an amount of deodorant and malodorous substance collected by the collecting surface, and the moving amount changing means may change the moving amount of the collecting surface in accordance with a collection amount calculated by the collection amount calculating means.

Further, the collection amount calculating means may detect an operating condition of a coating apparatus installed in the booth main body, and calculate the collection amount based on the operating condition.

Further, such a construction may be employed that the filter has a cylindrical filter base member for taking in a gas with malodor and deodorant from an outer periphery, and that the moving device rotates the filter base member in a circumferential direction thereof to move the collecting surface.

In the above constructions, in collecting and removing the deodorant and the malodorous substance by the filter, a collecting surface of high collection efficiency is successively moved toward the airflow (the inflow direction of the gas including the deodorant and the malodorous substance). The moving amount of the collecting surface can be adjusted according to the collection amount of deodorant and malodorous substance.

Here, the term "collection amount" covers both the amount of malodorous substance and deodorant collected by these apparatuses and the amount of malodorous substance and deodorant already collected. To suppress the malodor in the coating booth, the collection amount of deodorant and malodorous substance is obtained from the operating condition of the coating apparatus, and the moving amount of the collecting surface is changed according to the collection amount. Here, "the operating condition of the coating apparatus" can be grasped from various variables changing with coating operation, such ascoating materialejection amount, coating material ejection pressure, coating time, and compressed air consumption during coating.

Further, the deodorant supply device may include a supply amount adjusting means for adjusting a supply amount of deodorant, and a supply amount calculating means for calculating a deodorant supply amount corresponding to an amount of malodor to be deodorized, and the supply amount adjusting means may adjust the supply amount of deodorant in correspondence with the supply amount calculated by the supply amount calculating means.

Further, the supply amount calculating means may detect the operating condition of the coating apparatus, and, based on the operating condition, calculate the amount of deodorant to be supplied.

In the above constructions, the deodorant supply amount according to the amount of malodorous gas to be deodorized is calculated, and, based on the calculated supply amount, the deodorant supply amount is adjusted. When using this deodorization apparatus in a coating booth, the amount of malodorous gas to be deodorized is calculated from the operating condition of the coating apparatus installed in the coating booth. Further, such a construction may be employed that the airflow forming device includes a blower for forming an airflow, an air amount adjusting means for adjusting an air quantity of the blower, and a malodor amount calculating means for calculating an amount of malodor to be deodorized, and that the air quantity adjusting means adjusts the air quantity of the blower according to the malodor amount calculated by the malodor amount calculating means.

Further, the malodor amount calculating means may detect the operating condition of the coating apparatus, and calculate the malodor amount based on the operating condition.

In the above construction; the amount of malodorous gas to be deodorized is calculated by the malodorous gas amount calculating means, and, based on the malodorous gas amount calculated, the air quantity of the blower is adjusted. When using this deodorization apparatus in a coating booth, the amount of malodorous gas is calculated from the operating condition of the coating apparatus installed in the coating booth.

Further, a chemical filter is preferably used as the filter.

Further, the deodorization apparatus may include: a first casing unit containing the filter and the deodorant supply device and having the intake port at a position allowing introduction of the gas with malodor to a periphery of the deodorant supply device; and a second casing unit including the airflow forming device and the exhaust port and connected to the first casing unit through a filter provided in the first casing unit.

Further, a number of stages of the first casing unit may be connected to the second casing unit. Further, the deodorization apparatus may include a circulation duct which causes at least a part of a gas discharged from the second casing unit to flow back to the intake port of the first casing unit.

In the above constructions, the deodorization apparatus is formed by the first casing unit with deodorizing function and the second casing unit with airflow forming function. Thus, even if the capacity of the booth main body or the malodor generation amount is different, such situation can be easily coped with solely by changing the number of units forming the apparatus. Further, by causing the gas discharged from the second casing unit to flow back to the first casing unit, it is possible to keep the leakage of malodor to the exterior of the deodorization apparatus to a minimum.

Further, the present invention adopts the following deodorization method.

That is, the deodorization method is characterized by including: taking in a gas with malodor; supplying deodorant into the gas with malodor taken in to cause a malodorous substance generating malodor to adhere to the deodorant; and taking the gas with deodorant into a filter to collect the malodorous substance by the filter together with the deodorant.

Further, in the step of collecting the deodorant and the malodorous substance by the filter, the deodorant and the malodorous substance are collected while, of the collecting surface of the filter, a collecting surface with high collection efficiency is successively moved toward an airflow.

Further, in moving the collecting surface, a collection amount of deodorant and malodorous substance collected by the filter may be calculated, changing a moving amount of the collecting surface according to the collection amount calculated.

Further, when taking in malodor in a coating booth as a deodorization object, an operating condition of a coating device installed in the coating booth may be detected, calculating the collection amount of deodorant and malodorous substance based on the operating condition detected.

It is desirable that, in calculating the collection amount based on the operating condition of the coating device, the collection amount be calculated from an amount of compressed air consumed during application of coating material.

Further, in the step of supplying the deodorant, a supply amount of deodorant corresponding to an amount of malodor to be deodorized maybe calculated, adjusting the supply amount of deodorant according to the supply amount calculated.

Further, when taking in malodor in a coating booth as the object of deodorization, the operating condition of the coating device installed in the coating booth may be detected, calculating an amount of deodorant to be supplied based on the operating condition detected.

Further, in calculating a deodorant supply amount based on the operating condition of the coating device, an amount of deodorant to be supplied may be calculated from the amount of compressed air consumed during application of coating material.

Further, in the step of taking the gas with deodorant into the filter, an amount of deodorant and malodorous substance contained in the gas may be calculated, and an amount of gas to be taken into the filter may be adjusted according to the amount of deodorant and malodorous substance calculated.

Further, when taking in malodor in the coating booth as the object of deodorization, the operating condition of the coating device installed in the coating booth may be detected, calculating the amount of deodorant and malodorous substance contained in the gas based on the operating condition detected.

Further, in calculating the amount of deodorant and malodorous substance based on the operating condition of the coating device, the amount of deodorant and malodorous substance may be calculated from the amount of compressed air consumed during application of coating material.

According to the present invention, it is possible to provide a deodorization apparatus and a deodorization method enhanced in deodorizing efficiency and reduced in initial investment and running cost.

### BRIEF DESCRIPTION OF THE DRAWINGS

FIG. 1 is a side view of a coating booth according to an embodiment of the present invention.

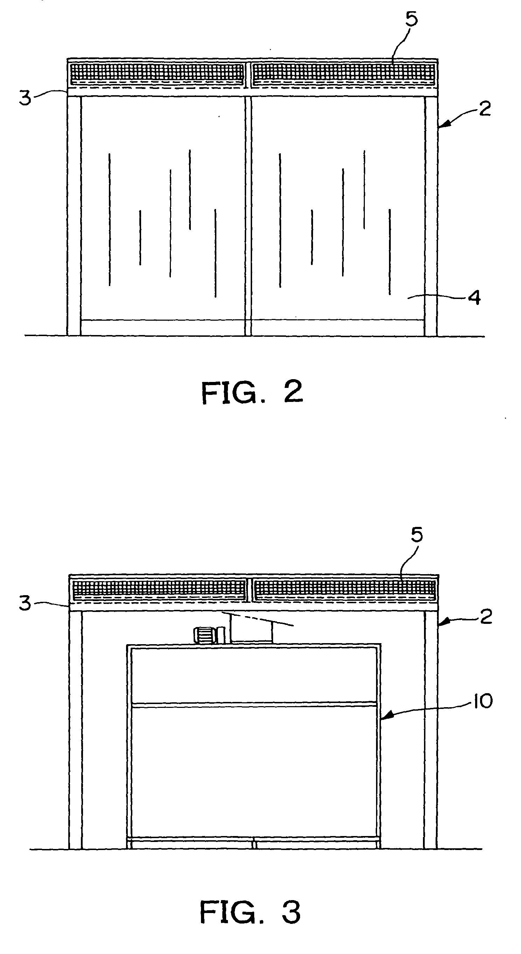

FIG. 2 is a front view of the coating booth of this embodiment.
FIG. 3 is a rear view of the coating booth of this embodiment.
FIG. 4 is a plan view of the coating booth of this embodiment.
FIG. 5 is a schematic view of a deodorization apparatus according to this embodiment.
FIG. 6 is a front view of the deodorization apparatus of this embodiment.
FIG. 7 is a diagram showing the internal construction of the deodorization apparatus of this embodiment.
FIG. 8 is a flowchart illustrating control procedures executed during operation of the deodorization apparatus.
FIG. 9 is a side view of a coating booth according to this embodiment which is equipped with a circulation duct for causing exhaust gas to flow back into the booth main body from the deodorization apparatus.
FIG. 10 is a diagram showing amodification of the deodorization apparatus of this embodiment.
FIG. 11 is a diagram showing a modi f ication of the deodorization apparatus of this embodiment.

### BEST MODE FOR CARRYING OUT THE INVENTION

In the following, a deodorization apparatus and a deodorization method according to an embodiment of the present invention will be described as applied to a coating booth. It should be noted that what is described with reference to this embodiment is only given by way of example, and that the following detailed specifications of the deodorization apparatus and the deodorization method of the present invention allow various modifications without departing from the technical scope of the invention as defined by the claims and are not restricted to the following description.

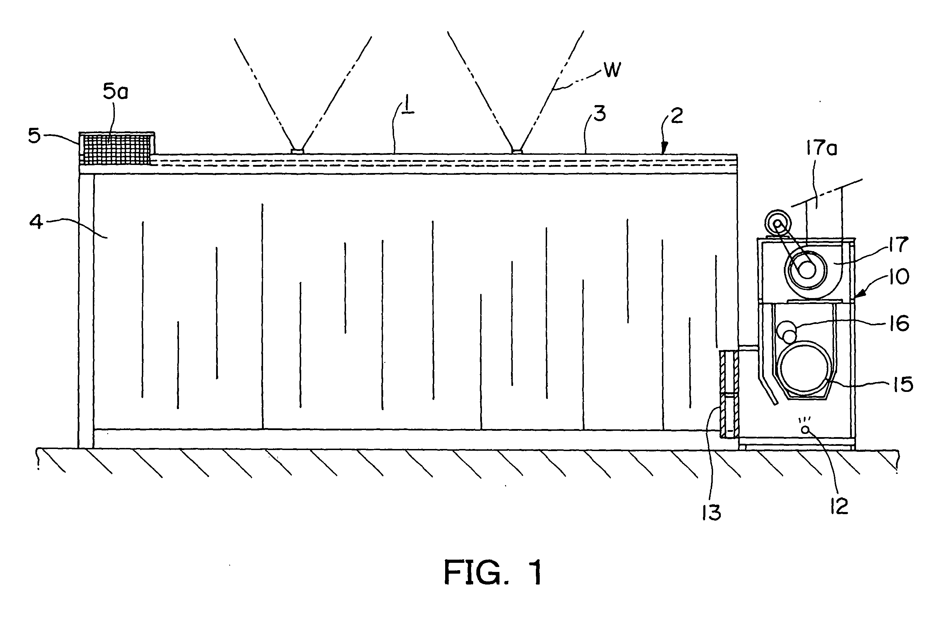

First, an outline of the coating booth of this embodiment will be described with reference to FIG. 1.

A booth 1 of this embodiment includes as main components a booth main body 2 restraining diffusion of malodor generated during operation, and a deodorization unit 10 for deodorizing malodor generated in the booth main body 2.

Further, in addition to these components, there are installed in the coating booth 1 coating devices needed for coating operation (e.g., an air compressor and a coating gun) (not shown).

The booth main body 2 is a so-called "indoor-installed type" plain booth, and includes a ceiling frame 3 suspended by wires W and a vinyl curtain 4 hanging down from the peripheral edge of the ceiling frame 3. The interior of the booth main body 2 is separated from the exterior by the curtain 4.

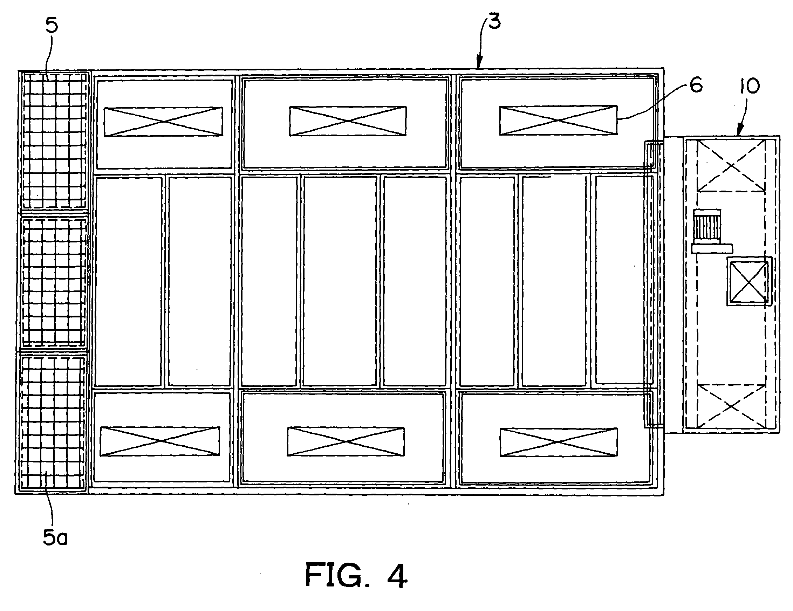

Further, as shown in Fig. 4, the ceiling frame 3 is equipped with an intake duct 5 and a plurality of fluorescent lamps 6.

The intake duct 5 serves to introduce fresh air (atmospheric air) into the booth main body 2 isolated from the atmospheric air. The intake duct 5 is provided on the upper surface side of the ceiling frame 3 and on the inlet side (the left-hand side in Fig. 1) of the booth main body 2. Further, a dust collecting filter 5a is mounted to the intake duct 5. The dust collecting filter 5a removes dust from air before the air is introduced into the booth main body 2 as fresh air. As shown in FIG. 4, the fluorescent lamps 6 are mounted to the lower surface of the ceiling frame 3.

Next, the deodorization apparatus 10 will be described.

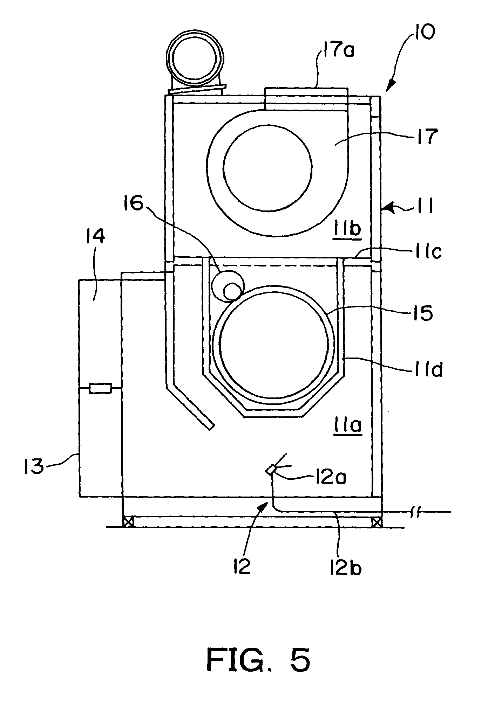

As shown in FIGS. 5 through 7, the deodorization apparatus 10 includes a casing 11 constituting the contour of the deodorization apparatus 10, a deodorant supply device 12 provided in the casing 11, an exhaust fan (airflow forming device) 13 forming an airflow in the casing 11, various filters 14 and 15 catching malodorous substance in the air flowing into the casing 11, and a main control unit (not shown) performing concentrated control on the various devices provided in the deodorization apparatus 10.

As shown in FIG. 5, the casing 11 can be divided into upper and lower stages by a partition wall 11c installed at the middle stage of the casing. In the following description, the casing forming the space of the lower stage of the casing (below the partition wall 11c) will be referred to as a first casing unit 11a. The casing forming the space of the upper stage of the casing (above the partition wall 11c) will be referred to as a second casing unit 11b.

First, the first casing unit 11a will be described.

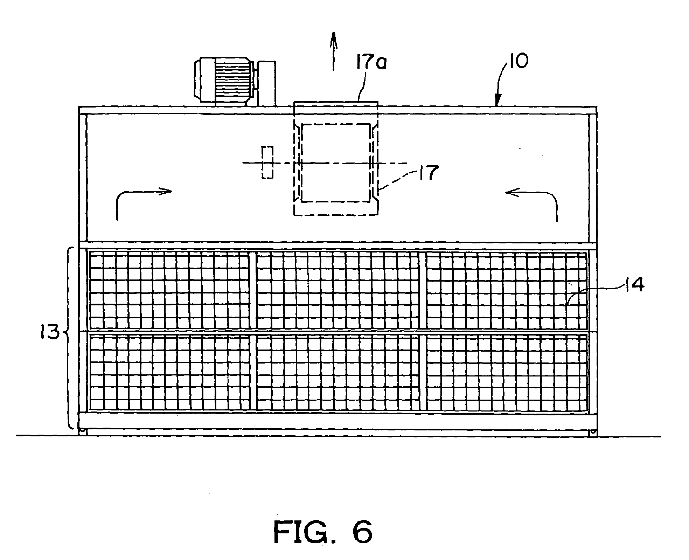

As shown in FIGS. 5 and 6, on the front side of the first casing unit 11a, there is formed the intake port 13. Further, the first casing unit 11a is connected to the interior of the booth main body 2 through the intake port 13. Malodor in the booth main body 2 is sucked into the first casing unit 11a through the intake port 13.

Further, the first filter 14 is mounted to the intake port 13.

The first filter 14 has a five-layer structure, and collects relatively large malodorous substance (e.g., coating mist) due to the physical properties of the filter base member thereof. Further, the first filter 14 is detachable from the intake port 13, and allows cleaning as needed.

Further, inside the first casing unit 11a, there are provided, as components of the deodorant supply device 12, injection nozzles 12a for injecting deodorant into the first casing unit 11a. In order to ensure uniform diffusion of the deodorant, six injection nozzles 12a in total are arranged at appropriate positions in the first casing unit 11a.

Further, the deodorant supply device 12 is equipped with injection amount adjusting devices (not shown) for adjusting the injection amount (deodorant supply amount) to an appropriate amount.

In this embodiment, as the injection amount adjusting devices, there are provided an electromagnetic valve adjusting the line pressure (supply pressure) of a deodorant supply line 12b leading to each of the injection nozzles 12a so as to change the injection amount, a pressure sensor monitoring the line pressure of the injection nozzle 12a, and a sub control unit (not shown) controlling the electromagnetic valve according to the deodorant supply amount calculated by the above-mentioned main control unit. In this embodiment, the injection amount adjusting devices and the above-mentioned main control unit constitute the supply amount adjusting means and the supply amount calculating means according to the claims of the invention.

In this embodiment, in calculating the deodorant supply amount in the main control unit, the malodor amounts (malodor generation amounts) at different times are calculated from the operating condition of the coating apparatus, and the requisite amounts of deodorant for deodorizing the malodor (malodor amount) as calculated are calculated.

More specifically, the consumption amount of compressed air consumed during coating operation (the pressure reduction amount in the air tank), the malodorous substance generation amount (the generation amount of coating mist or the like), and the requisite supply amount of deodorant for deodorizing the malodorous substance are related to each other through preliminary experiment, and the compressed air consumption amount at a particular in time is read first. Further, this compressed air consumption amount is converted to a malodorous substance generation amount. Then, a desired deodorant supply amount in correspondence with this generation amount is calculated. Then, the electromagnetic valve is controlled so as to attain an injection amount in conformity with the deodorant supply amount calculated.

In this way, in this embodiment, the requisite amounts of deodorant for deodorizing malodor at different times are supplied, whereby surplus consumption of deodorant is restrained.

Next, the partition wall 11c will be described.

The partition wall 11c corresponds to the top plate of the first casing unit 11a. Further, on either side of the partition wall 11c, there is provided a ventilation duct 11d extending from the inside of the first casing unit 11a to open in the upper end surface of the partition wall 11c. Further, the second filter 15, which is of a cylindrical configuration, is installed inside the first casing unit 11a so as to be supported at both ends by the lower ends of the ventilation ducts 11d.

Further, the interior of the second filter 15 communicates with the above-mentioned ventilation ducts 11d. The air within the first casing unit 11a is taken in from the outer peripheral portion of the second filter 15, and reaches the ventilation ducts 11d by way of the interior of the second filter 15. Then, it flows into the space above the partition wall 11c from the ventilation ducts 11d.

In this embodiment, as the second filter 15, a chemical filter is adopted which is superior in deodorant collecting/adsorbing capacity. The malodorous substance in the first casing unit 11a is collected by the second filter 15 together with the deodorant as it passes through the second filter 15. The second filter 15 can be freely extracted from the inside of the first casing unit 11a, and can be replaced when soiled to a certain degree.

Regarding the replacement of the second filter 15, the deodorization apparatus 10 of this embodiment is equipped with a pressure difference sensor (not shown) for detecting a difference in pressure between the upstream side and the downstream side of the second filter 15. Based on the level of pressure difference detected by the pressure difference sensor, the timing for replacement of the second filter 15 is ascertained.

Further, in connection with the illustration of the second filter 15, the deodorization apparatus 10 of this embodiment is equipped with a rotation drive device 16 for rotating the second filter 15.

The rotation drive device 16 includes a driving roller 16a held in contact with the outer periphery of the second filter 15 and adapted to rotate the same, a drive motor 16b for rotating the driving roller 16a, and a sub control unit for adjusting the RPM of the drive motor 16b according to the RPM of the drive motor 16b suitable for the collection amount of the malodorous substance and the deodorant at a particular point in time calculated by the above-mentioned main control unit.

Through rotation of the driving roller 16a, the second filter 15 is rotated, and a collecting surface with high collection efficiency is successively moved in the inflow direction (toward the airflow) of the gas containing the deodorant and the malodorous substance. Further, in this embodiment, the rotation drive device 16 and the above-mentioned main control unit constitute the moving device, moving amount changing means, and collection amount calculating means of the present invention.

As in the calculation of the deodorant supply amount described above, when calculating the collection amount of the malodorous substance and deodorant by the main control unit, the amount of compressed air consumed during coating is read first, and the generation amount of the malodor is obtained from the consumption amount of the compressed air. Then, regarding this generation amount as the collection amount of the malodorous substance and deodorant, the RPM of the drive motor 16b is set to a larger value as the collection amount increases. Thus, the second filter 15 rotates at an appropriate speed corresponding to the collection amount of the malodorous substance and deodorant at a particular point in time, so that local collection of the deodorant and malodorous substance with respect to the second filter 15 is restrained.

In this way, in the rotation drive device 16, when the amount of the deodorant and malodorous substance flowing into the second filter 15 is large, the RPM of the second filter 15 is increased. Further, a collecting surface with high collection efficiency (the upper surface side of the second filter) is successively moved toward the airflow side (which, in this embodiment, is mainly the lower surface side of the second filter 15).

Further, the rotation of the second filter 15 is also advantageous in enhancing the collection efficiency for the deodorant and malodorous substance. This is due to the fact that the deodorant and malodorous substance entering the filter base member of the second filter 15 receive a centrifugal force as a result of the rotation of the second filter 15, so that they remain within the filter base member without reaching the internal space of the second filter 15.

Next, the second casing unit 11b will be described.

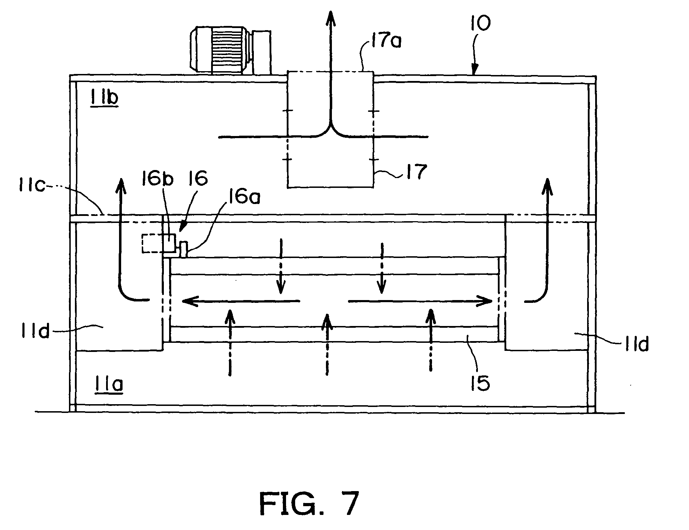

As shown in FIG. 7, the second casing unit 11b is connected to the first casing unit 11a below the partition wall 11c through the above-mentioned ventilation ducts 11d. Further, the second casing unit 11b is equipped with an exhaust fan 17 (blower) and an exhaust port 17a open to the atmosphere. Moreover, by the airflow formed by the exhaust fan 17, there is formed an airflow from the first casing unit 11a to the second casing unit 11b.

That is, during the operation of the exhaust fan 17, the air inside the booth main body 2 flows within the deodorization apparatus 10 in the following order: the booth main body 2, the intake port 13, the first casing unit 11a, the outer periphery of the second filter 15, the interior of the second filter 15, the ventilation ducts 11d, the second casing unit 11b, the exhaust fan 17, and the exhaust port 17a.

Further, the exhaust fan 17 is provided with an air quantity adjusting device (not shown). This air quantity adjusting device adjusts the air quantity of the exhaust fan 17 based on a control value calculated by the above-mentioned main control unit in order to maintain the deodorization efficiency in the coating booth 1 at a fixed level. That is, when the malodor amount is large, the deodorization efficiency is relatively low. Accordingly, so that the air quantity of the exhaust fan 17 is set relatively high, with the malodor amount being large or increasing in order to increase the intake air amount, thereby restraining the deterioration in the deodorization efficiency. Further, in this embodiment, the air quantity adjusting device and the main control unit constitute the air quantity adjusting means and malodor amount calculating means according to the claims of the present invention.

As in the case of the above-described injection amount adjusting device, in adjusting the air quantity, the malodor amount in the booth main body 2 is calculated from the amount of compressed air consumed at the time of injection of the coating material, and based on the malodor amount thus calculated, the exhaust fan 17 is controlled so as to provide an output in conformity with the malodor amount at a particular point in time.

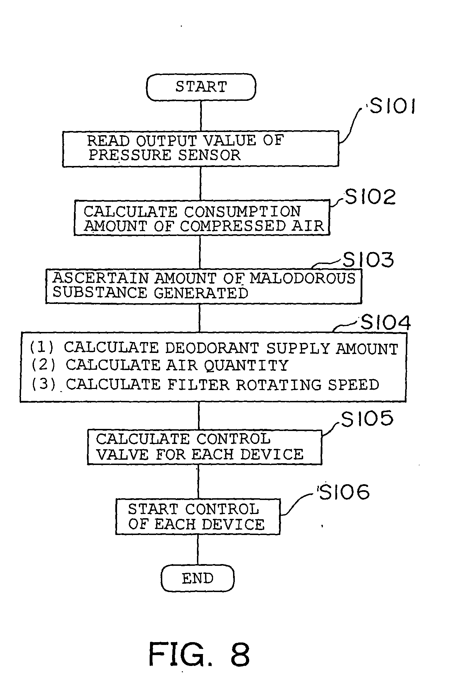

FIG. 8 is a flowchart illustrating the procedures executed in the main control unit during the operation of the deodorization apparatus 10. In the following, various control procedures will be illustrated with reference to this flowchart.

First, with the start of coating operation, the main control unit reads the output of a pressure sensor provided in an air compressor (S101). The pressure sensor serves to detect the pressure in the air tank, that is, the amount of compressed air in the air tank. In this step, the output value of this pressure sensor is read each time a predetermined period of time elapses, monitoring the pressure in the tank at different times during the coating operation.

Subsequently, in the main control unit, the output change amount, that is, the pressure change amount, at each point in time from the output value read in step 101, and the consumption amount of the compressed air is calculated through conversion from that pressure change amount (S102).

Further, in the main control unit, after the processing of step 102, in order to calculate the requisite control value for the supply amount control in the injection amount adjusting device 12 (electromagnetic valve control), the air quantity control in the air quantity adjusting device (the output control of the exhaust fan 1), and the RPM control in the rotation drive device 16 (RPM control of the drive motor 16b), the coating material injection amount, that is, the generation amount of the malodorous substance in the booth main body 2, is calculated based on the consumption amount of the compressed air calculated in step 102 (S103).

Moreover, based on the malodor amount thus calculated, the deodorant supply amount, the air quantity, and the filter rotating speed are calculated (S104), and based on the calculated values, the control values for the respective devices are obtained (S105). The requisite collection amount of deodorant for the RPM control of the second filter 15 is grasped from the deodorant supply amount calculated in step 104. The control values are then transmitted to the sub control unit provided in the injection amount adjusting device 12, the air quantity adjusting device, and the sub control unit provided in the rotation drive device 16,thereby controlling the various devices through the sub control unit or directly (S106).

In this way, in the coating booth 1 of this embodiment, air with malodor (the air in the booth main body 2) is first taken in the first casing unit 11a through the first filter 14, collecting relatively large coating mist by the first filter. Next, deodorant is injected into that air to cause adhesion of the residual malodorous substance, such as fine coating mist and vaporized solvent, to the deodorant. After the residual malodorous substance is collected by the second filter 15 together with the deodorant, the air is discharged from the deodorization apparatus 10.

That is, the deodorization apparatus 10 effects both sufficient deodorization with deodorant even in a small injection amount, and collection by the filter, whereby it is possible to maintain the deodorizing capacity by a simple maintenance operation involving no water treatment, such as filter replacement or filter cleaning. Further, through application of the deodorization apparatus 10, there is no need to provide large-scale equipments, such as a vessel and a contaminated water processing device.

The deodorization apparatus 10, in which deodorant is supplied instead of water and which requires maintenance of the second filter 15, may seem to incur an increase in running cost. However, it provides various advantages: (1) the deodorant, which has a higher deodorizing capacity as compared with water, provides a sufficient deodorizing effect with a small supply amount (injection amount) ; (2) it requires no contaminated water processing by using a contaminated water processing device; and (3) it requires no periodical replacement of water. Thus, compared with the conventional deodorization apparatus, it may be said that the deodorization apparatus 10 can be maintained at a much lower running cost.

It should be noted that the above-described embodiment is given by way of example only, and the present invention allows specific modifications as needed.

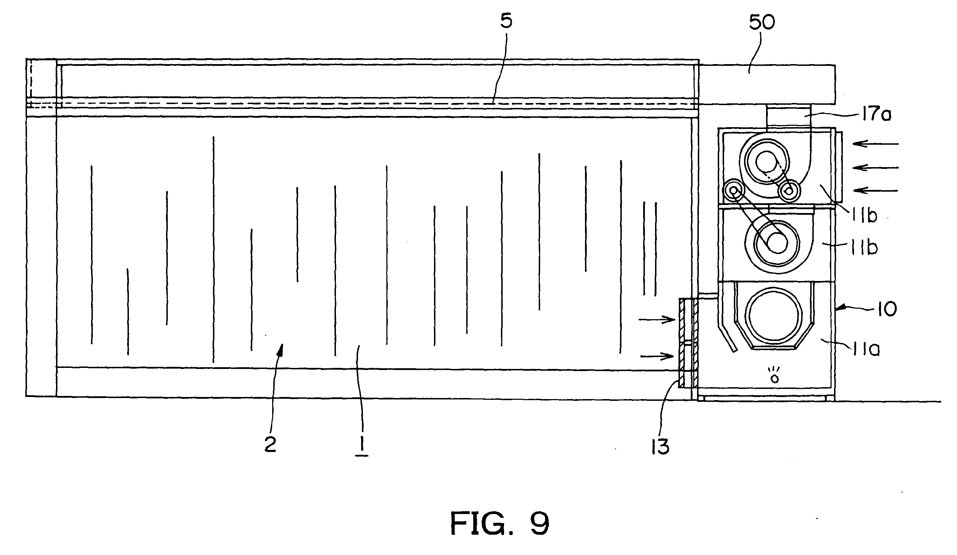

For example, while in the above-described apparatus the exhaust port 17a is open to the atmosphere, and the deodorized air is discharged into the atmosphere, it is also possible, as shown in FIG. 9, to provide a circulation duct 50 establishing communication from the exhaust port 17a to the above-mentioned intake duct 5, thereby re-circulating the air in the deodorization apparatus 10 into the booth main body 2 through the circulation duct 50. In this case, the adhesion of the deodorant and malodorous substance to the second filter 15 is effected more reliably.

Further, while in the above-described embodiment the requisite supply amount of the deodorant for deodorization of the malodor and the malodor amount in the coating booth 1 are calculated from the amount of compressed air consumed during injection of the coating material, it is also possible to calculate them from the operating amount of the coating gun (e.g., the needle stroke amount), the coating time, and the like.

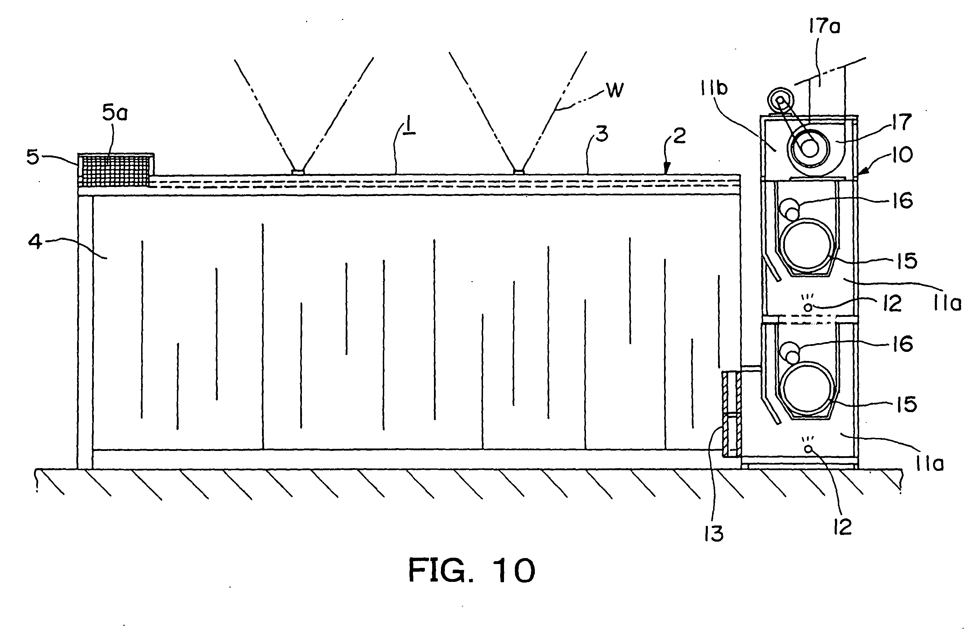

Further, while in the above-described deodorization apparatus 10 the air with the deodorant is purified by a single second filter 15, it is also possible, for example, to adopt a construction in which a number of first casing units 11a each equipped with a second filter 15 are superimposed one upon the other and connected to the second filter 15 (see FIG. 10), that is, to install a plurality of second filters 15. Further, this embodiment employs a casing 11 that can be separated by the partition wall 11c, so that, by increasing the number of casings constituting the first casing unit 11a, the number of second filters 15 can be easily increased. That is, the specifications of the deodorization apparatus 10 can be easily changed according to the kind of operation, and the like performed in each coating booth 1.

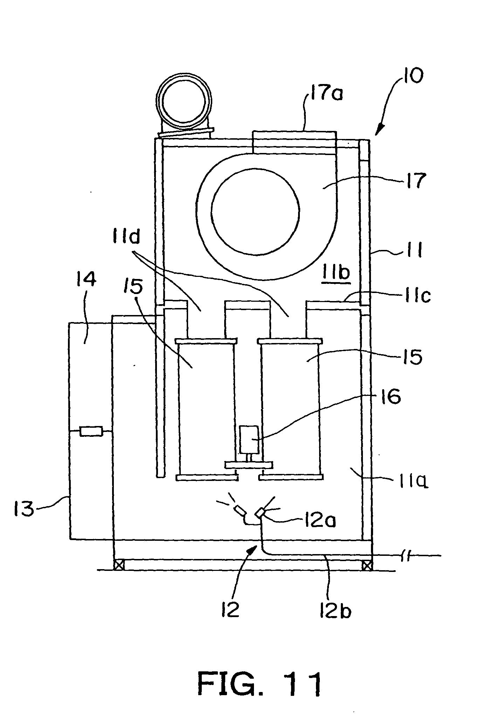

Further, as shown in FIG. 11, it is also possible to adopt modifications such as mounting second filters 15 in a vertical position and installing a plurality of them in the first casing unit 11a. Further, while in the above-described embodiment the collecting surface is moved by rotating the second filter 15 itself, it is also possible to provide a current plate in the vicinity of the second filter 15, changing the air inflow direction with respect to the second filter 15 by means of this current plate. Further, it is also possible to provide a cover covering the collecting surface of the second filter 15 and to provide a slit serving as an air intake port in a part of this cover, causing the collecting surface to make relative movement by moving the cover itself.

## Claims

1. A deodorization apparatus **characterized by** comprising:
an intake port for taking in a gas with malodor;
a deodorant supply device for supplying deodorant into the gas taken in through the intake port;
an exhaust port for discharging the gas taken in through the intake port;
an airflow forming device for forming an airflow from the intake port to the exhaust port; and
a filter which removes a malodorous substance from the gas with malodor together with the deodorant prior to exhaust through the exhaust port.

2. A deodorization apparatus according to Claim 1, **characterized in that** the deodorization apparatus comprises a booth main body for restraining diffusion of malodor, and that an intake port is open to an interior of the booth main body.

3. A deodorization apparatus according to Claim 1 or 2, **characterized by** comprising a moving device for moving a collecting surface of the filter for collecting the deodorant and the malodorous substance.

4. A deodorization apparatus according to Claim 3, **characterized in that**, in moving the collecting surface, the moving device moves a collecting surface with a high collection efficiency toward the airflow.

5. A deodorization apparatus according to Claim 3 or 4, **characterized in that**:
the moving device comprises a moving amount changing means for changing amoving amount of the collecting surface and a collection amount calculating means for calculating an amount of deodorant and malodorous substance collected by the collecting surface; and
the moving amount changing means changes the moving amount of the collecting surface in accordance with a collection amount calculated by the collection amount calculating means.

6. A deodorization apparatus according to Claim 5, **characterized in that** the collection amount calculating means detects an operating condition of a coating apparatus installed in the booth main body, and calculates the collection amount based on the operating condition.

7. A deodorization apparatus according to any one of Claims 3 through 6, **characterized in that**:
the filter has a cylindrical filter base member for taking in a gas with malodor and deodorant from an outer periphery; and
the moving device rotates the filter base member in a circumferential direction thereof to move the collecting surface.

8. A deodorization apparatus according to any one of Claims 1 through 7, **characterized in that**:
the deodorant supply device comprises a supply amount adjusting means for adjusting a supply amount of deodorant, and a supply amount calculating means for calculating a deodorant supply amount corresponding to an amount of malodor to be deodorized; and
the supply amount adjusting means adjusts the supply amount of deodorant in correspondence with the supply amount calculated by the supply amount calculating means.

9. A deodorization apparatus according to Claim 8, **characterized in that** the supply amount calculating means detects the operating condition of the coating apparatus, and, based on the operating condition, calculates the amount of deodorant to be supplied.

10. A deodorization apparatus according to any one of Claims 1 through 9, **characterized in that**:
the airflow forming device comprises a blower for forming an airflow, an air amount adjusting means for adjusting an air quantity of the blower, and a malodor amount calculating means for calculating an amount of malodor to be deodorized; and
the air quantity adjusting means adjusts the air quantity of the blower according to the malodor amount calculated by the malodor amount calculating means.

11. A deodorization apparatus according to Claim 10, **characterized in that** the malodor amount calculating means detects the operating condition of the coating apparatus, and calculates the malodor amount based on the operating condition.

12. A deodorization apparatus according to any one of Claims 1 through 11, **characterized in that** the filter is a chemical filter.

13. A deodorization apparatus according to any one of Claims 1 through 12, **characterized in that** the deodorization apparatus comprises:
a first casing unit containing the filter and the deodorant supply device and having the intake port at a position allowing introduction of the gas with malodor to a periphery of the deodorant supply device; and
a second casing unit comprising the airflow forming device and the exhaust port and connected to the first casing unit through a filter provided in the first casing unit.

14. A deodorization apparatus according to Claim 13, **characterized in that** a number of stages of the first casing unit are connected to the second casing unit.

15. A deodorization apparatus according to Claim 13 or 14, **characterized by** comprising a circulation duct which causes at least a part of a gas discharged from the second casing unit to flow back to the intake port of the first casing unit.

16. A deodorization method **characterized by** the steps of:
taking in a gas with malodor;
supplying deodorant into the gas with malodor taken in to cause a malodorous substance generatingmalodor to adhere to the deodorant; and
taking the gas with deodorant into a filter to collect the malodorous substance by the filter together with the deodorant.

17. A deodorization method according to Claim 16, **characterized in that**, in the step of collecting the deodorant and the malodorous substance by the filter, the deodorant and the malodorous substance are collected while, of the collecting surface of the filter, a collecting surface with high collection efficiency is successively moved toward an airflow.

18. A deodorization method according to Claim 17, **characterized in that**, in moving a collecting surface, the collection amount of deodorant and malodorous substance collected by the filter is calculated, changing a moving amount of the collecting surface according to the collection amount calculated.

19. A deodorization method according to Claim 18, **characterized in that**, when taking in malodor in a coating booth as a deodorization object, an operating condition of a coating device installed in the coating booth is detected, calculating the collection amount of deodorant and malodorous substance based on the operating condition detected.

20. A deodorization method according to Claim 19, **characterized in that**, in calculating the collection amount based on the operating condition of the coating device, the collection amount is calculated from an amount of compressed air consumed during application of coating material.

21. A deodorization method according to any one of Claims 16 to 20, **characterized in that**, in the step of supplying the deodorant, a supply amount of deodorant corresponding to an amount of malodor to be deodorized is calculated, adjusting the supply amount of deodorant according to the supply amount calculated.

22. A deodorization method according to Claim 21, **characterized in that**, when taking in malodor in a coating booth as the object of deodorization, the operating condition of the coating device installed in the coating booth is detected, calculating an amount of deodorant to be suppliedbased on the operating condition detected.

23. A deodorization method according to Claim 22, **characterized in that**, in calculating a deodorant supply amount based on the operating condition of the coating device, the amount of deodorant to be supplied is calculated from the amount of compressed air consumed during application of coating material.

24. A deodorization method according to any one of Claims 16 to 23, **characterized in that**, in the step of taking the gas with deodorant into the filter, an amount of deodorant and malodorous substance contained in the gas is calculated, and an amount of gas to be taken into the filter is adjusted according to the amount of deodorant and malodorous substance calculated.

25. A deodorization method according to Claim 2 4, **characterized in that**, when taking in malodor in the coating booth as the object of deodorization, the operating condition of the coating device installed in the coating booth is detected, calculating the amount of deodorant and malodorous substance contained in the gas based on the operating condition detected.

26. A deodoriz at ion method according to Claim 25, **characterized in that**, in calculating the amount of deodorant and malodorous substance based on the operating condition of the coating device, the amount of deodorant and malodorous substance is calculated from the amount of compressed air consumed during application of coating material.
